# FASCICULE DE BREVET EUROPEEN

(11) **EP 1 000 542 B1**
(45) Date de publication et mention de la délivrance du brevet: **12.06.2002**
(21) Numéro de dépôt: 99402569.0
(22) Date de dépôt: 18.10.1999
(51) Int. Cl.: A01N 31/02, A61K 7/48

(54) **Système à activité antimicrobienne et son utilisation, notamment dans les domaines cosmétique et dermatologique**
Antimikrobielle Zusammensetzung und deren Verwendung, insbesondere in Kosmetika und Dermatologie
Antimicrobial composition and its use, especially in cosmetics and dermatology

(30) Priorité: 09.11.1998 FR 9814077
(43) Date de publication de la demande: 17.05.2000
(73) Titulaire: L'OREAL, 75008 Paris (FR)
(72) Inventeur: Cupferman, Sylvie, 94240 L'Hay Les Roses (FR); Marion, Catherine, 92330 Sceaux (FR)
(74) Mandataire: Rasson, Catherine

(56) Documents cités:
- EP-A- 0 572 271
- FR-A- 2 729 050
- US-A- 3 970 759
- DATABASE WPI Derwent Publications Ltd., London, GB; AN 76-72925X XP002108494 KAO SOAP CO LTD: "Non-medical bacteriostatic for foods, cosmetics- comprising 1,2 diols contg 8-12 carbons" & JP 51 091327 A
- DATABASE WPI Derwent Publications Ltd., London, GB; AN 97-128621 XP002108495 HOKU KK: "aq. compsn. for cosmetic and hair growth agent, etc.- comprises hinokitiol or its salts and urea" & JP 09 012423 A
- DATABASE CAPLUS [Online] 1987 YOSHIKOSHI, AKIRA ET AL.: "Preparation of hinokitiol as an antibacterial and antifungal agent" retrieved from STN, accession no. 1987:576270 Database accession no. 107:176270 XP002108492 & JP 62 093250 A
- DATABASE CAPLUS [Online] 1982 POLA CHEMICAL INDUSTRIES: "cosmetic preservation with dihydric alcohols" retrieved from STN, accession no. 1982:129596 Database accession no. 96:129596 XP002108491 & JP 57 002212 A

## Description

La présente invention se rapporte à un nouveau système antimicrobien comprenant (I) au moins un polyol, en association avec (II) le cycloheptatriène-2,4,6-one-1 hydroxy-2 (méthyl-1 éthyl)-4 ou le capryl-lactyl-lactylate de sodium, et les compositions, en particulier cosmétiques, dermatologiques, pharmaceutiques ou alimentaires, contenant un tel système dans un support physiologiquement acceptable. L'invention se rapporte aussi à l'utilisation du dit système comme agent antimicrobien dans une composition cosmétique ou dermatologique.

Il est courant d'introduire dans les compositions cosmétiques ou dermatologiques des conservateurs chimiques destinés à lutter contre le développement des micro-organismes dans ces compositions, qui les rendraient rapidement inaptes à l'utilisation. Il faut protéger les compositions à la fois contre les micro-organismes susceptibles de se développer à l'intérieur de la composition et contre ceux que l'utilisateur peut introduire dans celle-ci en la manipulant, et en particulier lors de la préhension avec les doigts de produits en pot.

Des conservateurs chimiques couramment utilisés sont notamment les parabens ou des donneurs de formol. Ces conservateurs présentent l'inconvénient de causer des intolérances telles que irritations et/ou allergies, et en particulier sur les peaux sensibles. Il en est de même pour les alcools ou les polyols, tels que l'éthanol ou le propylène glycol, en particulier lorsqu'ils sont présents à des taux relativement importants, c'est-à-dire en des quantités excédant 20 % en poids par rapport à l'ensemble de la composition.

Le document JP-A-51 091327 décrit l'utilisation de 1,2-diols comportant de 8 à 12 atomes de carbones comme agents bactériostatiques. D'autre part, le document EP-572 271 décrit l'association de butane-1,3-diol et de sodium capryl/ sodium lauryl lactylate (1 :1) pour son activité antimoisissure.Par ailleurs, le document FR-A-2,747,572 décrit l'utilisation d'alcanes-1,2-diols pour inactiver les micro-organismes cutanés. Toutefois, ces composés aux concentrations habituelles d'utilisation ne permettent pas de couvrir tout le spectre microbien, ou bien il est nécessaire de les introduire en des quantités telles que l'innocuité et la tolérance cutanée des produits finis ne sont plus satisfaisantes.

En outre, le document FR-A-2,729,050 décrit l'association d'un polyol en C₂-C₈ avec au moins un mono-(C₃ - C₉)-alkyl ou (C₃ - C₉)-alcényléther de glycérol et ne contenant pas plus de 15% de monoalcool(s) aliphatique(s). Cependant, la quantité de polyols nécessaire pour obtenir une activité antimicrobienne à large spectre n'est pas compatible avec l'ensemble des formulations cosmétiques et, surtout, peut créer des problèmes d'irritation cutanée.

On constate donc que subsiste le besoin d'agents antimicrobiens ayant une action au moins aussi efficace que les composés de l'art antérieur, mais ne présentant pas leurs inconvénients.

Ainsi, après de nombreuses recherches menées sur la question, la demanderesse a maintenant découvert, de façon inattendue et surprenante, qu'un système comprenant au moins un polyol particulier associé au cycloheptatriène-2,4,6-one-1 hydroxy-2 (méthyl-1 éthyl)-4 ou au capryl-lactyl-lactylate de sodium, présentait intrinsèquement une action antimicrobienne importante et synergique, permettant d'assurer, par sa seule présence, la conservation des compositions dans lesquelles il est introduit.

Cette association est avantageuse, car elle ne provoque pas d'intolérance et/ou de rougeur lors de l'application sur la peau d'une composition cosmétique ou dermatologique comprenant cette association, tout en présentant une activité antimicrobienne au moins aussi efficace que celle des composés de l'art antérieur.

La découverte de ces sytèmes antimicrobiens est à la base de la présente invention.

Par antimicrobien, on entend selon l'invention un système résistant à, ou permettant de lutter contre, la dégradation microbienne et ayant donc une activité antibactérienne et/ou une activité antifongique et/ou une activité antivirale.

La présente invention a ainsi pour objet un système antimicrobien, caractérisé par le fait qu'il comprend (I) au moins un polyol choisi parmi les polyols comportant de 4 à 8 atomes de carbone et les mono-(C₃-C₉)alkyl- ou (C₃-C₉)alcényl-éthers de glycérol, et (II) le cycloheptatriène-2,4,6-one-1 hydroxy-2 (méthyl-1 éthyl)-4.

Parmi les polyols comportant de 4 à 8 atomes de carbone, on utilise de préférence ceux comportant de 5 à 8 atomes de carbone tels que l'octane diol-1,2, le pentane diol-1,2, l'isoprène glycol et le sorbitol. Comme mono-(C₃-C₉)alkyl- ou (C₃-C₉)alcényl-éthers de glycérol, on peut citer par exemple l'octoxyglycérine (ou 3-[(2-ethylhexyl)oxy]-1,2-propanediol), le 3-[(heptyl)oxy]-1,2-propanediol, le 3-[(octyl)oxy]-1,2-propanediol et le 3-[(allyl)oxy]-1,2-propanediol, ou un mélange de deux ou plusieurs de ces polyols. On utilise de manière préférée l'octane diol-1,2, le pentane diol-1,2 et/ou l'octoxyglycérine.

Le cycloheptatriène-2,4,6-one-1 hydroxy-2 (méthyl-1 éthyl)-4 (nom CTFA : Hinokitiol) peut être synthétisé ou être obtenu à partir de végétaux et par exemple à partir d'huile extraite d'arbres, telles que l'huile d'Hiba ou d'huile de cèdre. On peut utiliser aussi les sels du cycloheptatriène-2,4,6-one-1 hydroxy-2 (méthyl-1 éthyl)-4 et notamment les sels de métal alcalin (sodium, potassium), alcalino-terreux (magnésium) ou de bases organiques (diéthanolamine).

La présente invention a aussi pour objet un système antimicrobien, caractérisé par le fait qu'il comprend (I) au moins un polyol choisi parmi l'octane diol-1,2, le pentane diol-1,2, l'isoprène glycol, le sorbitol et les mono-(C₃-C₉)alkyl- ou (C₃-C₉)alcényl-éthers de glycérol, et (II) le capryl-lactyl-lactylate de sodium.

Le capryl-lactyl-lactylate de sodium (nom CTFA : sodium caproyl lactylate) est le sel de sodium de l'ester caprylique du lactyl-lactate.

Les mono-(C₃-C₉)alkyl- ou (C₃-C₉)alcényl-éthers de glycérol peuvent être notamment ceux indiqués ci-dessus.

Dans les systèmes indiqués ci-dessus, le rapport pondéral du polyol (I) par rapport au composé (II) peut varier dans une large mesure. Il va de préférence de 0,1 à 20000, plus particulièrement de 0,25 à 100.

Les systèmes antimicrobiens synergiques de l'invention présentent notamment l'avantage d'être efficaces sur différents germes, et notamment à la fois sur *Enterococus faecalis* et sur *Candida albicans*. En outre, dans ces systèmes, le polyol peut être présent dans une composition à des quantités plus faibles que lorsqu'il est utilisé seul.

Ces systèmes sont plus particulièrement destinés à un usage cosmétique, pharmaceutique, dermatologique ou alimentaire. Quand ils sont destinés à une application par voie topique telle que cosmétique ou dermatologique, ils comprennent alors un support physiologiquement acceptable, c'est-à-dire compatible avec la peau, les cheveux et/ou les muqueuses.

Un autre objet de l'invention est l'utilisation des systèmes définis ci-dessus en tant qu'agent antimicrobien dans une composition cosmétique ou dermatologique.

La présente invention a aussi pour objet une composition comprenant, dans un support physiologiquement acceptable, au moins un système antimicrobien tel que défini ci-dessus.

Selon un mode préféré de réalisation de l'invention, la composition selon l'invention est exempte d'agents conservateurs classiques.

Les compositions comprenant les systèmes antimicrobiens de l'invention peuvent contenir par exemple de 0,01 à 20 % et mieux de 0,1 à 5 % en poids de polyol(s) (I) par rapport au poids total de la composition et de 0,001 à 10 % et mieux de 0,01 à 5 % en poids de composé(s) (II) par rapport au poids total de la composition.

Les compositions de l'invention, dans le cas d'une application cosmétique ou dermatologique, peuvent comprendre en outre les adjuvants cosmétiques et/ou dermatologiques classiquement mis en oeuvre comme les corps gras, les solvants organiques, les épaississants ioniques ou non ioniques, les adoucissants, les antioxydants, les opacifiants, les stabilisants, les agents anti-mousse, les agents hydratants, les vitamines, les parfums, les émulsifiants ioniques ou non, les charges, les séquestrants, les électrolytes, les colorants ou tout autre ingrédient habituellement utilisé en cosmétique ou dermatologie. Les quantités des différents adjuvants des compositions selon l'invention sont celles classiquement utilisées dans les domaines considérés. La nature des adjuvants et leurs quantités doivent être telles qu'elles ne modifient pas les propriétés des compositions selon l'invention.

Les compositions selon l'invention peuvent se présenter sous forme de solution, de suspension ou de dispersion dans des solvants tels que l'eau ou des corps gras, sous forme de dispersion vésiculaire ou encore sous forme d'émulsion eau-dans-huile (E/H), huile-dans-eau (H/E) ou multiple, telle qu'une crème ou un lait, sous forme de pommade, de gel, de bâtonnet solide, de mousse aérosol ou de spray.

Lorsque la composition est une émulsion, la proportion de la phase grasse peut aller de 5 à 80 % en poids, et de préférence de 5 à 50 % en poids par rapport au poids total de la composition. Les huiles, les cires, les émulsionnants et les coémulsionnants utilisés dans la composition sous forme d'émulsion sont choisis parmi ceux classiquement utilisés dans les domaines cosmétique et dermatologique. L'émulsionnant et le coémulsionnant sont présents, dans la composition, en une proportion allant de préférence de 0,3 à 30 % en poids, et mieux de 0,5 à 20 % en poids par rapport au poids total de la composition. L'émulsion peut, en outre, contenir des vésicules lipidiques.

Comme corps gras, on peut utiliser par exemple les huiles d'origine végétale, les huiles de silicone volatiles (cyclométhicone) ou non volatiles, les huiles minérales (vaseline, isoparaffine), les huiles de synthèse et notamment les esters gras, les alcools gras (alcool cétylique), les acides gras et les cires.

Comme émulsifiants, on peut utiliser tous ceux compatibles avec le système de l'invention. Dans le cas d'une émulsion E/H, on peut utiliser par exemple un émulsifiant siliconé tel que le cetyl dimethicone copolyol, et dans le cas d'une émulsion H/E, on peut utiliser par exemple un ou plusieurs esters d'acide gras, tels que le stéarate de glycéryle (nom CTFA : glyceryl stearate), le stéarate de polyéthylène glycol à 40 groupes oxyéthylénés (nom CTFA : PEG-40 stearate), le tristéarate de sorbitan (nom CTFA : sorbitan tristearate) et le stéarate de sodium (nom CTFA : sodium stearate).

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium.

Les compositions selon l'invention sont préparées selon les techniques bien connues de l'homme de l'art du domaine.

Dans tout ce qui suit ou ce qui précède, les pourcentages sont donnés en poids sauf mention contraire.

D'autre caractéristiques et avantages de l'invention pourront apparaître dans les exemples qui suivent, qui sont donnés à titre purement illustratif et nullement limitatif. Les noms des composés sont donnés selon le cas en nom CTFA ou en nom chimique.

### Exemple 1 : émulsion E/H

- Acetylated glycol stearate / tristearin
   (UNITWIX de la société GUARDIAN) 0,12 %
- Magnesium sulfate 0,175 %
- Polyacrylamide / C13-14 Isoparaffin/Laureth-7
   (Sepigel 305 de la société SEPPIC) 1 %
- Cetyl dimethicone copolyol
   (ABIL EM 90 de la société GOLDSCHMIDT) 0,75 %
- Huile de silicone 12 %
- Octane diol-1,2 0,25 %
- Capryl-lactyl-lactylate de sodium 1 %
- Eau qsp 100 %

**Exemple comparatif 1** : la composition est identique à celle de l'exemple 1 mais sans capryl-lactyl-lactylate de sodium.

**Exemple comparatif 1'** : la composition est identique à celle de l'exemple 1 mais sans octane diol-1,2.

### Exemple 2 : émulsion E/H

- Acetylated glycol stéarate / tristearin
   (UNITWIX de la société GUARDIAN) 0,12 %
- Magnesium sulfate 0,175 %
- Polyacrylamide / C13-14 lsoparaffin/Laureth-7
   (Sepigel 305 de la société SEPPIC) 1 %
- Cetyl dimethicone copolyol
   (ABIL EM 90 de la société GOLDSCHMIDT) 0,75 %
- Huile de silicone 12 %
- Octane diol-1,2 0,25 %
- Hinokitiol 0,01 %
- Eau qsp 100 %

**Exemple comparatif 2** : la composition est identique à celle de l'exemple 2 mais sans octane diol-1,2.

### Exemple 3 : émulsion E/H

- Acetylated glycol stearate / tristearin
   (UNITWIX de la société GUARDIAN) 0,12 %
- Magnesium sulfate 0,175 %
- Polyacrylamide / C13-14 lsoparaffin/Laureth-7
   (Sepigel 305 de la société SEPPIC) 1 %
- Cetyl dimethicone copolyol
   (ABIL EM 90 de la société GOLDSCHMIDT) 0,75 %
- Huile de silicone 12 %
- Pentane diol-1,2 3 %
- Capryl-lactyl-lactylate de sodium 1 %
- Eau qsp 100 %

**Exemple comparatif 3** : la composition est identique à celle de l'exemple 3 mais sans capryl-lactyl-lactylate de sodium.

**Exemple comparatif 3'** : la composition est identique à celle de l'exemple 3 mais sans pentane diol-1,2.

### Exemple 4 : émulsion E/H

- Acetylated glycol stearate / tristearin
   (UNITWIX de la société GUARDIAN) 0,12 %
- Magnesium sulfate 0,175 %
- Polyacrylamide / C13-14 Isoparaffin/Laureth-7
   (Sepigel 305 de la société SEPPIC) 1 %
- Cetyl dimethicone copolyol
   (ABIL EM 90 de la société GOLDSCHMIDT) 0,75 %
- Huile de silicone 12 %
- Pentane diol-1,2 3 %
- Hinokitiol 0,01 %
- Eau qsp 100 %

**Exemple comparatif 4'** : la composition est identique à celle de l'exemple 4 mais sans pentane diol-1,2.

### Exemple 5 : émulsion huile-dans-eau (H/E)

- Sorbitan tristearate 0,9 %
- Huile de vaseline 4 %
- Isoparaffine 10,5 %
- Cyclométhicone 5 %
- PEG-40 stearate 2 %
- Alcool cétylique 4 %
- Glyceryl stearate (TEGIN de la société GOLDSCHMIDT) 3 %
- Sodium stearate 1,2 %
- Octane diol-1,2 0,25 %
- Capryl-lactyl-lactylate de sodium 1 %
- Eau qsp 100 %

**Exemple comparatif 5** : la composition est identique à celle de l'exemple 5 mais sans capryl-lactyl-lactylate de sodium.

**Exemple comparatif 5'** : la composition est identique à celle de l'exemple 5 mais sans octane diol-1,2.

### Exemple 6 : émulsion H/E

- Sorbitan tristearate 0,9 %
- Huile de vaseline 4 %
- Isoparaffine 10,5 %
- Cyclométhicone 5 %
- PEG-40 stearate 2 %
- Alcool cétylique 4 %
- Glyceryl stearate (TEGIN de la société GOLDSCHMIDT) 3 %
- Sodium stearate 1,2 %
- Octane diol-1,2 0,25 %
- Hinokitiol 0,01 %
- Eau qsp 100 %

**Exemple comparatif 6** : la composition est identique à celle de l'exemple 6 mais sans octane diol-1,2.

### Exemple 7 : émulsion H/E

- Sorbitan tristearate 0,9 %
- Huile de vaseline 4 %
- Isoparaffine 10,5 %
- Cyclométhicone 5 %
- PEG-40 stearate 2 %
- Alcool cétylique 4 %
- Glyceryl stearate (TEGIN de la société GOLDSCHMIDT) 3 %
- Sodium stearate 1,2 %
- Pentane diol-1,2 3 %
- Capryl-lactyl-lactylate de sodium 1 %
- Eau qsp 100 %

**Exemple comparatif 7** : la composition est identique à celle de l'exemple 7 mais sans capryl-lactyl-lactylate de sodium.

**Exemple comparatif 7'** : la composition est identique à celle de l'exemple 7 mais sans pentane diol-1,2.

### Exemple 8 : émulsion H/E

- Sorbitan tristearate 0,9 %
- Huile de vaseline 4 %
- Isoparaffine 10,5 %
- Cyclométhicone 5 %
- PEG-40 stearate 2 %
- Alcool cétylique 4 %
- Glyceryle stearate (TEGIN de la société GOLDSCHMIDT) 3 %
- Sodium stearate 1,2 %
- Pentane diol-1,2 3 %
- Hinokitiol 0,01 %
- Eau qsp 100 %

**Exemple comparatif 8** : la composition est identique à celle de l'exemple 8 mais sans pentane diol-1,2.

**Mise en évidence de l'activité antimicrobienne du système revendiqué :**

Le test suivant met en évidence l'activité antimicrobienne du système selon l'invention.

### 1. Matériel

a) Micro-organismes
   Bactérie : *Enterococcus faecalis* (American Type Culture Collection N° 33186), cultivé sur Trypcase soja
   Levure : *Candida albicans* (American Type Culture Collection N°10231), cultivé sur Sabouraud.
b) Milieux de culture
   Préparation inoculum : diluant tryptone-sel
   Dénombrements : bouillon et gélose Eugon LT100
c) Appareillage
   Equipement classique d'un laboratoire de microbiologie.

### 2. Méthode

1. La veille du test, on repique chaque souche microbienne sur le milieu gélosé approprié et on met à incuber à l'étuve à 35°C.
2. Le jour du test, on pèse 20 grammes du produit dans un pilulier stérile. On inocule à 1% à l'aide d'une suspension microbienne titrant à 10⁸ germes par ml : on obtient une concentration de 10⁶ dans le produit.
   On met à incuber l'échantillon contaminé à l'étuve à 22°C.
3. Après 7 jours d'incubation, on reprend 1 gramme de produit, on effectue des dilutions décimales dans le bouillon Eugon LT100 et on étale ces dilutions à la surface de boîtes de Pétri contenant de la gélose Eugon LT100. On met les boîtes à incuber à 35°C.
4. On procède au comptage des colonies sur les boîtes contenant plus de 20 et moins de 200 colonies.

### 3. Résultats

Le protocole décrit ci-dessus a été appliqué aux compositions des exemples 1 à 8 renfermant le système de l'invention et aux compositions des exemples comparatifs ainsi qu'à une composition ne comportant aucun des composés du système de l'invention (placebo).

Les résultats obtenus sont repris dans les tableaux ci dessous. Ils sont exprimés en nombre de micro-organismes par gramme de préparation, restant dans le produit après 7 jours de contact.

Ces tests mettent en évidence l'existence d'une synergie entre les deux polyols testés et le capryl-lactyl-lactylate de sodium et l'hinokitiol. L'utilisation dans une formulation cosmétique simultanément d'un polyol et d'un des trois composés précités permet d'obtenir une protection vis-à-vis des bactéries et des fungi sans rencontrer les problèmes de tolérance cutanée liés à l'utilisation de conservateurs chimiques traditionnels ou à un taux élevé de polyols.

**Tableau 1**

| Germes | Placebo | Ex. 1 | Ex. comparatif 1 | Ex. comparatif 1' | Ex. 2 | Ex. comparatif 2 |
|---|---|---|---|---|---|---|
| *Entero-coccus faecalis* | 10⁶ | < 200 | 10⁴ | 10³ | 10⁴ | 10⁵ |
| *Candida albicans* | 10⁶ | <200 | 10⁵ | 10⁶ | <200 | 10⁶ |

**Tableau 2**

| Germes | Placebo | Ex. 3 | Ex. comparatif 3 | Ex. comparatif 3' | Ex. 4 | Ex. comparatif 4 |
|---|---|---|---|---|---|---|
| *Entero-coccus faecalis* | 10⁶ | <200 | 10⁵ | 10³ | < 200 | 10⁵ |
| *Candida albicans* | 10⁶ | <200 | 10⁶ | 10⁶ | <200 | 10⁶ |

**Tableau 3**

| Germes | Placebo | Ex. 5 | Ex. comparatif 5 | Ex. comparatif 5' | Ex. 6 | Ex. comparatif 6 |
|---|---|---|---|---|---|---|
| *Entero-coccus faecalis* | 10⁶ | < 200 | 3,7. 10⁵ | 10⁴ | < 200 | 10⁶ |
| *Candida albicans* | 10⁶ | <200 | 10⁶ | 10⁶ | <200 | 10⁵ |

**Tableau 4**

| Germes | Placebo | Ex. 7 | Ex. comparatif 7 | Ex. comparatif 7' | Ex. 8 | Ex. comparatif 8 |
|---|---|---|---|---|---|---|
| *Entero-coccus faecalis* | 10⁶ | <200 | 2,3.10⁵ | 10⁴ | <200 | 10⁶ |
| *Candida albicans* | 10⁶ | <200 | 10⁶ | 10⁶ | <200 | 10⁵ |

Ces tableaux montrent un synergie entre le polyol (octane diol-1,2 ou pentane diol-1,2) et le composé associé dans tous les cas pour la résistance à *Candida albicans* et dans pratiquement tous les cas pour la résistance à *Enterococcus faecalis.*

Par ailleurs, le tableau 5 montre que le capryl-lactyl-lactylate de sodium présente une synergie avec les polyols revendiqué et pas avec les autres et notamment pas avec le butylène glycol (ou butanediol) :

**Tableau 5**

| Germes | Capryl-lactyl-lactylate de sodium (1 %) | Octane-diol (0,25%) | Pentane-diol (3 %) | Butane-diol (3 %) | Capryl-lactyl-lactylate de sodium (1%) + octane-diol (0,25%) | Capryl-lactyl-lactylate de sodium (1%) + pentane-diol (3%) | Capryl-lactyl-lactylate de sodium (1%) + butane-diol (3%) |
|---|---|---|---|---|---|---|---|
| *Entero- coccus faecalis* | 1.5.10⁴ | 3.7.10⁵ | 2.3.10⁵ | 4.0.10⁵ | <200 | <200 | 6.8.10³ |

En microbiologie, deux valeurs sont considérées comme significativement diffréentes lorsque leur écart est supérieur à 0,5 Log. Ainsi, 6.8.10³ n'est pas significativement différent de 1,5.104 et il n'y a donc pas de synergie entre le capryl-lactyl-lactylate de sodium et le butanediol.

## Revendications

1. Système antimicrobien, **caractérisé par le fait qu'**il comprend (I) au moins un polyol choisi parmi les polyols comportant de 4 à 8 atomes de carbone et les mono-(C₃-C₉)alkyl- ou (C₃-C₉)alcényl-éthers de glycérol, et (II) le cycloheptatriène-2,4,6-one-1 hydroxy-2 (méthyl-1 éthyl)-4.

2. Système selon la revendication 1, **caractérisé en ce que** le polyol (I) est choisi parmi l'octane diol-1,2, le pentane diol-1,2, l'isoprène glycol, le sorbitol, l'octoxyglycérine, le 3-[(heptyl)oxy]-1,2-propanediol, le 3-[(octyl)oxy]-1,2-propanediol, le 3-[(allyl)oxy]-1,2-propanediol et leurs mélanges.

3. Système antimicrobien, **caractérisé par le fait qu'**il comprend (I) au moins un polyol choisi parmi l'octane diol-1,2, le pentane diol-1,2, le sorbitol et les mono-(C₃-C₉)alkyl- ou (C₃-C₉)alcényl-éthers de glycérot, et (II) le capryl-lactyl-lactylate de sodium.

4. Système selon l'une quelconque des revendications 1 à 3, **caractérisé en ce que** le rapport pondéral du polyol (I) par rapport au composé (II) va de 0,1 à 20000.

5. Composition comprenant, dans un support physiologiquement acceptable, au moins un système antimicrobien selon l'une quelconque des revendications 1 à 4.

6. Composition selon la revendication 5, **caractérisée en ce qu'**elle comprend de 0,01 à 20 % en poids de polyol(s) (I) par rapport au poids total de la composition.

7. Composition selon la revendication 5 ou 6, **caractérisée en ce qu'**elle comprend de 0,001 à 10 % en poids de composé(s) (II) par rapport au poids total de la composition.

8. Composition selon l'une quelconque des revendications 5 à 7, **caractérisée en ce qu'**elle est destinée à un usage topique, de préférence cosmétique ou dermatologique.

9. Composition selon l'une quelconque des revendications 5 à 8, **caractérisée en ce qu'**elle est sous forme d'une émulsion.

10. Utilisation du système selon l'une quelconque des revendications 1 à 4 en tant qu'agent antimicrobien dans une composition cosmétique ou dermatologique.

## Patentansprüche

1. Antimikrobielles System, **dadurch gekennzeichnet, daß** es (I) mindestens ein Polyol, das unter den Polyolen mit 4 bis 8 Kohlenstoffatomen und den Mono-C₃₋₉-alkyl- oder -C₃₋₉-alkenylethern von Glycerin ausgewählt ist, und (II) 2-Hydroxy-4-(1-methyl-ethyl)-2,4,6-cycloheptatrien-1-on enthält.

2. System nach Anspruch 1, **dadurch gekennzeichnet, daß** das Polyol (I) unter 1,2-Octandiol, 1,2-Pentandiol, Isoprenglykol, Sorbit, Octoxyglycerin, 3-[(Heptyl)oxy]-1,2-propandiol, 3-[(Octyl)oxy]-1,2-propandiol und 3-[(Allyl)oxy]-1,2-propandiol und deren Gemischen ausgewählt ist.

3. Antimikrobielles System, **dadurch gekennzeichnet, daß** es (I) mindestens ein Polyol, das unter 1,2-Octandiol, 1,2-Pentandiol, Sorbit und den Mono-C₃₋₉-alkyl- oder Mono-C₃₋₉-alkenylethem von Glycerin ausgewählt ist, und (II) Natriumcapryllactyllactat enthält.

4. System nach einem der Ansprüche 1 bis 3, **dadurch gekennzeichnet, daß** das Gewichtsverhältnis von Polyol (I) und Verbindung (II) im Bereich von 0,1 bis 20 000 liegt.

5. Zusammensetzung, die in einem physiologisch akzeptablen Träger ein antimikrobielles System nach einem der Ansprüche 1 bis 4 enthält.

6. Zusammensetzung nach Anspruch 5, **dadurch gekennzeichnet, daß** sie 0,01 bis 20 Gew.-% Polyol(e) (I), bezogen auf das Gesamtgewicht der Zusammensetzung, enthält.

7. Zusammensetzung nach Anspruch 5 oder 6, **dadurch gekennzeichnet, daß** sie 0,001 bis 10 Gew.-% Verbindung(en) (II), bezogen auf das Gesamtgewicht der Zusammensetzung, enthält.

8. Zusammensetzung nach einem der Ansprüche 5 bis 7, **dadurch gekennzeichnet, daß** sie vorzugsweise für die Kosmetik und Dermatologie zur topischen Anwendung vorgesehen ist.

9. Zusammensetzung nach einem der Ansprüche 5 bis 8, **dadurch gekennzeichnet, daß** sie in Form einer Emulsion vorliegt.

10. Verwendung des Systems nach einem der Ansprüche 1 bis 4 als antimikrobieller Wirkstoff in einer kosmetischen oder dermatologischen Zusammensetzung.

## Claims

1. Antimicrobial system, **characterized in that** it comprises (I) at least one polyol chosen from polyols comprising from 4 to 8 carbon atoms and mono (C₃-C₉) alkyl or (C₃-C₉)alkenyl glyceryl ethers and (II) 2-hydroxy-4-(1-methylethyl)cyclohepta-2,4,6-trien-1-one.

2. System according to Claim 1, **characterized in that** the polyol (I) is chosen from 1,2-octanediol, 1,2-pentanediol, isoprene glycol, sorbitol, octoxyglycerol, 3-[(heptyl)oxy]-1,2-propanediol, 3-[(octyl)oxy]-1,2-propanediol and 3-[(allyl)oxy]-1,2-propanediol and mixtures thereof.

3. Antimicrobial system, **characterized in that** it comprises (I) at least one polyol chosen from 1,2-octanediol, 1,2-pentanediol, sorbitol and mono(C₃-C₉)alkyl or (C₃-C₉)alkenyl glyceryl ethers and (II) sodium capryl lactyl lactylate.

4. System according to any one of Claims 1 to 3, **characterized in that** the weight ratio of the polyol (I) relative to the compound (II) ranges from 0.1 to 20,000.

5. Composition comprising, in a physiologically acceptable support, at least one antimicrobial system according to any one of Claims 1 to 4.

6. Composition according to Claim 5, **characterized in that** it comprises from 0.01 to 20% by weight of polyol(s) (I) relative to the total weight of the composition.

7. Composition according to Claim 5 or 6, **characterized in that** it comprises from 0.001 to 10% by weight of compound(s) (II) relative to the total weight of the composition.

8. Composition according to any one of Claims 5 to 7, **characterized in that** it is intended for topical use, preferably cosmetic or dermatological use.

9. Composition according to any one of Claims 5 to 8, **characterized in that** it is in the form of an emulsion.

10. Use of the system according to any one of Claims 1 to 4 as an antimicrobial agent in a cosmetic or dermatological composition.
